# EUROPEAN PATENT APPLICATION

(11) **EP 2 143 433 A1**
(43) Date of publication of application: **13.01.2010**
(21) Application number: 09162002.1
(22) Date of filing: 05.06.2009
(51) Int. Cl.: A61K 31/728, A61P 19/02

(54) **Preparation for the prevention of joint disease**

(30) Priority: 08.07.2008 CZ 200820122 U
(71) Applicant: ISOline s.r.o., 11000 Praha 1 (CZ)
(72) Inventor: Micka, Robert, 58001, Havlíckúv Brod (CZ)
(74) Representative: Novotny, Jaroslav

(57) **Abstract**

According to this invention, the above-mentioned disadvantages are removed by the product for prevention of joint disease, the basis of which is the content of 10 to 1,000 mg of hyaluronic acid and/or salts from it in 100 to 1,000 grams of the medicine's carrier. The advantage of the product for the prevention of joint diseases is that it has an instant ingredient or sugar in the carrier. The advantage of the product for the prevention of joint diseases is that the carrier is formed by a liquid. The advantage of the product for the prevention of joint diseases is that it contains a mixture in the carrier for the production of snack sticks.

## Description

### TECHNICAL FIELDS

The invention concerns a preparation for the prevention of joint diseases, with the active ingredient reaching the body automatically with customary drinking or diet and its dosage does not, therefore, depend on the memory of the individual.

### BACKGROUND ARTS

The preparations for the prevention of joint diseases are available so far as food supplements in various latent states, most often as capsules or syrups. A person who intends to use such products must carry them all the time and use them with drinks. This causes considerable problems mainly in manual professions, e.g. for construction workers. It happens sometimes that some individuals feel ashamed to use drugs publicly and it is difficult for them to take these preparations during breaks or lunch. This results in postponing the use of these preparations to a later date, usually the evening, when the preparation is not completely absorbed and utilized by the body. There are also preparations with hyaluronic acid, such as those mentioned e.g. in WO 2005/032276, for which a molecular mass of 500,000 to 4,000,000 g/mol is given. A nutritional drink is also known according to WO 2006/070453, which contains hyaluronic acid in addition to other components. Another product, according to US 2005/0084518, contains a combination of hyaluronic acid with another active ingredient. Low molecular mass hyaluronic acid is also contained in a food supplement according to EP 1 707 578. WO 2004/089098 also indicates a combination of hyaluronic acid with a milk product. The drug fighting joint disease according to EP 1 354 590 also indicates the use of hyaluronic acid. The content of this acid is also mentioned in WO 2004/004686. The use of hyaluronic acid for the treatment of inflammatory processes is also discussed by WO 02/069984. The active ingredient - hyaluronic acid - is also mentioned in PV 2003-262. The situation is similar in PV 2001-3110, in which hyaluronic acid is in a complex with zinc.

However, the disadvantage of all these products is that they are indicated as a medicinal product with the need for regular use. This encounters problems of remembering the time of regular use and it is also necessary to have this drug or product available within reach all the time. Such a product is, therefore, not suitable for use in the field. Some of these products are combined with other substances, which are also present in other food and therefore could overload the body with these additional substances.

Another method for transporting the required products to the joints is by injection. However, in this case the situation is even worse, since many patients with potential joint disease do not want to visit a doctor, because an injection is a relatively painful and long procedure.

### DISCLOSURE OF INVENTION

According to this invention, the above-mentioned disadvantages are removed by the product for prevention of joint disease, the basis of which is the content of 10 to 1,000 mg of hyaluronic acid and/or salts from it in 100 to 1,000 grams of the medicine's carrier.

The advantage of the product for the prevention of joint diseases is that it has an instant ingredient or sugar in the carrier.

The advantage of the product for the prevention of joint diseases is that the carrier is formed by a liquid.

The advantage of the product for the prevention of joint diseases is that it contains a mixture in the carrier for the production of snack sticks.

According to this invention, the advantage of the product for the prevention of joint diseases is that it is distributed automatically from a machine for the preparation of coffee, chocolate, tea or other drinks, because it is a part of the instant mixture for the preparation of the drink. If the active ingredient is present in the liquid, it enters the body by drinking these beverages, contained usually in cans, boxes or PET containers. The potential user does not need to think about swallowing capsules with the active ingredient; each time he/she drinks the beverage, he/she may be sure that the active ingredient is contained in it. This automatically helps to create the awareness of a regular drinking regime at the site equipped with drinking machines, where the person is present or prepares the drink from an instant mixture containing the active ingredient. The active ingredient also reaches the body in a natural manner during the consumption of snack sticks.

### MADE FOR CARRING OUT THE INVENTION

1. The preparation for the prevention of joint diseases consists in 10 mg potassium hyaluronate contained in 100 mg of instant drink.
2. The preparation for the prevention of joint diseases consists in 100 mg potassium hyaluronate contained in 100 mg of instant drink.
3. The preparation for the prevention of joint diseases consists in 500 mg magnesium hyaluronate contained in 100 mg of instant drink.
4. The preparation for the prevention of joint diseases consists in 800 mg sodium hyaluronate contained in 100 ml of liquid drink.
5. The preparation for the prevention of joint diseases consists in 1,000 mg sodium hyaluronate contained in 1,000 mg of sugar.
6. The preparation for the prevention of joint diseases consists in 20 mg magnesium hyaluronate contained in 40 g of mixture for the production of snack sticks.

The recommended dose of the active ingredient is 7 to 20 mg/day, which is fulfilled e.g. by sugar with 1,000 ml of active substance/1,000 mg of sugar, which means 2.5 to 5 g of sugar per 150 ml of coffee.

The instant drink is prepared from a 20 g bag in a quantity of 1.5 litres of drink containing 20 mg of the active substance.
40 g of the stick mixture contains 20 mg of the active substance.

## Claims

1. The preparation for the prevention of joint diseases is **characterized by** a content of 10 to 1,000 mg of hyaluronic acid and/or salts from it in 100 to 1,000 g of the carrier.

2. The preparation for the prevention of joint diseases according to claim 1 is **characterized by** the instant ingredient or sugar in the carrier.

3. The preparation for the prevention of joint diseases according to claim 1 is **characterized by** a carrier consisting in fluid.

4. The preparation for the prevention of joint diseases according to claim 1 is **characterized by** a carrier consisting in a mixture for the production of snack sticks.

## Amended claims

### Amended claims in accordance with Rule 137(2) EPC.

**1.** The preparation for the prevention of joint diseases which contains from 10 to 1000 mg of hyaluronic acid and/or salts from it is **characterized in that** is contained in 100 to 1000 g of the carrier.

**2.** The preparation according to claim 1 is **characterized in that** the carrier which is made of the instant ingredient or sugar.

**3.** The preparation according to claim 1 is **characterized in that** a carrier consisting in fluid.
